# EUROPEAN PATENT SPECIFICATION

(11) **EP 2 594 187 B1**
(45) Date of publication and mention of the grant of the patent: **30.03.2016**
(21) Application number: 11835991.8
(22) Date of filing: 30.09.2011
(51) Int. Cl.: A61B 1/00, A61B 1/06, G02B 23/26

(54) **ENDOSCOPE**
ENDOSKOP
ENDOSCOPE

(30) Priority: 25.10.2010 JP 2010238800
(43) Date of publication of application: 22.05.2013
(73) Proprietor: Olympus Corporation, Shibuya-ku Tokyo (JP)
(72) Inventor: OUE, Takeshi, Tokyo 151-0072 (JP); KUDO, Akira, Tokyo 151-0072 (JP); SATO, Yosuke, Tokyo 151-0072 (JP)
(74) Representative: Schicker, Silvia
(86) International application number: PCT/JP2011/072552
(87) International publication number: WO 2012/056851

(56) References cited:
- WO-A1-2006/001377
- WO-A1-2006/046559
- WO-A1-2007/018098
- JP-A- 2002 051 971
- JP-A- 2004 248 835
- JP-A- 2005 027 851
- JP-A- 2007 007 322
- JP-A- 2009 022 588
- US-A1- 2007 173 695
- US-A1- 2007 191 684
- US-A1- 2008 128 740
- US-B1- 6 796 939

## Description

### BACKGROUND OF THE INVENTION

### 1. Field of the Invention

The present invention relates to an endoscope including a light-emitting device installed at a distal end portion of an insertion portion, as an illumination optical system.

### 2. Description of the Related Art

Endoscopes are widely used in a medical field and an industrial field. Objects to be examined or observed by endoscopes are inner portions of, e.g., living bodies or plants. Thus, when an endoscopic observation is performed, a light source that illuminates an object to be observed is required.

A general endoscope apparatus includes an endoscope, and a light source apparatus as an outside apparatus for the endoscope. Illuminating light emitted by the light source apparatus is supplied to a light guide provided in the endoscope. The supplied illuminating light is conveyed by the light guide and is emitted from an illuminating window arranged at a distal end of an insertion portion of the endoscope to illuminate an object to be observed.

In recent years, endoscopes including a light-emitting device, such as a light-emitting diode, provided at a distal end portion of an insertion portion, instead of a combination of a light source apparatus and light guide fibers, to directly illuminate an object to be observed with light emitted by the light-emitting device have been proposed.

For example, Japanese Patent Application Laid-Open Publication No. 2004-248835 (hereinafter referred to as document 1) discloses an endoscope that prevents a decrease in an amount of illuminating light or generation of image noise due to heat emitted by an LED illumination (corresponding to a light-emitting device in the present invention) arranged at a distal end portion of an insertion portion, thereby enabling favorable observation to be performed over a long period of time. In the endoscope, a heat dissipation member that conducts heat generated from the light-emitting device arranged at the distal end portion of the insertion portion to the rear side of the insertion portion is provided to cool the light-emitting device.

However, in document 1 the heat dissipation member is a bundle wire member obtained by bundling a plurality of strands each formed of a material having high thermal conductivity and having a diameter not greater than 0.1mm. The bundle wire member is arranged on an inner circumference side distant from a central position of a space inside the insertion portion. Therefore, when bending a bending portion provided in the insertion portion, the bundle wire member in the bending portion is also bent in accordance with bending motion thereof. Then, by repeatedly performing the bending motion of the bending portion, the bundle wire member is also repeatedly bent so that the strands constituting the bundle wire member are cut off to cause a fear of exerting undesirable influence to heat dissipativity.

US 6,796,939 B1 discloses an electronic endoscope with an illuminating section in which an LED for illumination that emits illuminating light to illuminate an observed site is provided. The illuminating section further comprises an LED substrate at which a heat sink may be provided.

US 2008/0128740 A1 discloses an endoscope with a light emitting unit, wherein the light-emitting unit comprises at least one electrode member having high thermal conductivity and low resistance, and a flip chip type light-emitting device electrically connected to the at least one electrode member. Heat generated in the light-emitting device is released along the longitudinal direction of the electrode member. A mesh-like heat sink member may be fixed and thermally connected to the at least one electrode member.

US 2007/0191684 A1 discloses an endoscope with an insertion portion and an LED adaptor, wherein the LED adaptor has a plurality of LED chips, an LED supporting block that is provided on the LED adaptor and that supports the plurality of LED chips, and a heat removal portion that removes heat from the LED supporting block. In an embodiment, the heat removal portion comprises a heat discharge portion that is inserted into a throughhole formed in the LED supporting block and that is in contact with an insertion portion.

US 2007/0173695 A1 discloses another embodiment of an endoscope device in which at least a portion of an objective lens group is mounted on an LED supporting block, which also supports LED chips of an illumination device.

The present invention has been made in view of the above circumstances and an object of the present invention is to provide an endoscope capable of preventing a decrease in an amount of illuminating light or generation of image noise to thereby enable favorable observation to be performed over a long period of time by dissipating heat generated from a light-emitting device arranged at a distal end portion of an endoscope.

### SUMMARY OF THE INVENTION

The above object is achieved with the features of independent claim 1.

An endoscope according to an aspect of the present invention includes: a distal end rigid member made of a metal and provided on a distal end side of a bending portion, an observation optical portion being fixedly provided in the distal end rigid member; a distal end cover formed of an insulating resin member and fixed to the distal end rigid member in an integrated manner, the distal end cover including a transparent illuminating window portion included in an illumination optical portion and a light-emitting device installing hole; a light-emitting device arranged in the light-emitting device installing hole, the light-emitting device including a light-emitting portion that faces the illuminating window portion included in the distal end cover and is fixed at a predetermined position, and a conductive portion positioned on the illuminating window portion side relative to an opening of the light-emitting device installing hole, the conductive portion being opposed to the opening of the light-emitting device installing hole; a substrate having thermal conductance and combining as a heat dissipating member, the substrate being connected to the conductive portion of the light-emitting device, and a power supply cable that supplies power being connected to the substrate; and a heat dissipation member having a plate shape and thermal conductance, an end side of the heat dissipation member being connected to the substrate and another end side of the heat dissipation member projecting relative to the substrate to dissipate heat conducted to the substrate.

### BRIEF DESCRIPTION OF THE DRAWINGS

Fig. 1 is a diagram illustrating a distal end face of an insertion portion of an endoscope;
Fig. 2 is a cross-sectional diagram along line Y2-Y2 of Fig. 1 and is a diagram illustrating a configuration of the distal end portion of the endoscope;
Fig. 3 is a cross-sectional diagram illustrating a configuration of a light-emitting device and a distal end cover;
Fig. 4 is a cross-sectional diagram illustrating a configuration of a distal end rigid member;
Fig. 5 is a diagram illustrating concave-channel-shaped heat dissipation plate including a flexed portion;
Fig. 6 is a cross-sectional diagram illustrating a configuration of a distal end portion of an endoscope including a heat dissipation plate that is the concave-channel-shaped heat dissipation plate in Fig. 5 with a flat braided conductive wire fixed to the flexed portion;
Fig. 7 is a cross-sectional diagram illustrating a relationship between a cover unit having a heat dissipation function and a distal end rigid member;
Fig. 8A is a cross-sectional diagram illustrating a distal end cover including an illuminating window portion that includes an exit face having an arc-like convex shape portion;
Fig. 8B is a cross-sectional diagram illustrating a distal end cover including an illuminating window portion that includes an exit face having an arc-like concave shape portion;
Fig. 9A is a cross-sectional diagram illustrating a distal end cover in which an illuminating window portion includes a transparent first resin member and portions other than the illuminating window portion include a second resin member that constitutes a light-blocking portion;
Fig. 9B is a diagram illustrating a distal end cover in which a portion between an observation optical portion and an illumination optical portion includes a second resin member that constitutes a light-blocking portion and all of other portions include a transparent first resin member; and
Fig. 9C is a diagram illustrating a distal end cover in which the illumination optical portion side of a portion between an observation optical portion and an illumination optical portion includes a second resin member that provides a light-blocking portion and all of other portions include a transparent first resin member.

### DETAILED DESCRIPTION OF THE PREFERRED EMBODIMENT

Hereinafter, an embodiment of the present invention will be described with reference to the drawings.

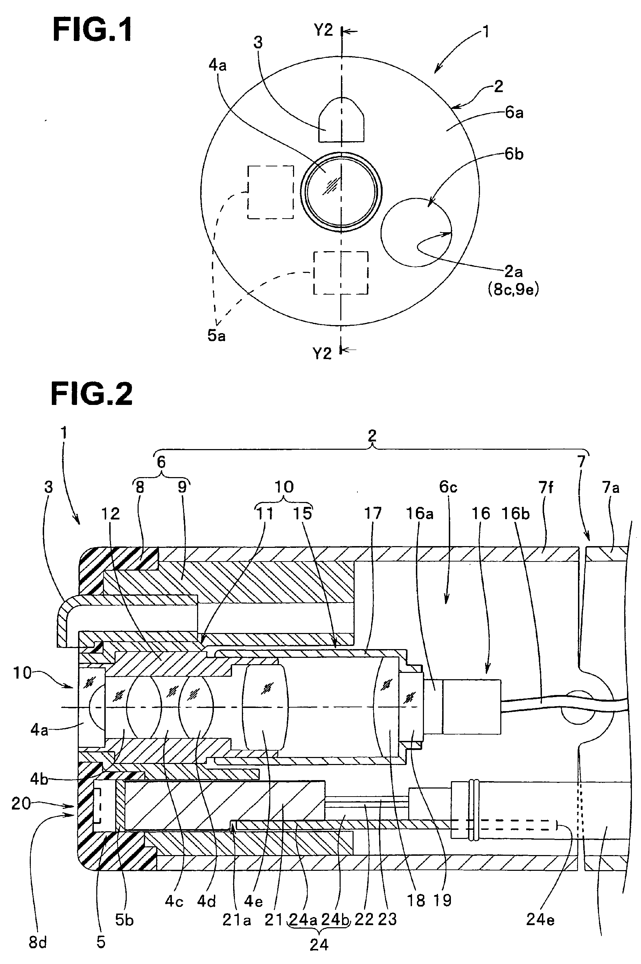

As illustrated in Figs. 1 and 2, an endoscope 1 according to the present embodiment includes an elongated insertion portion 2. At a distal end face 6a of the endoscope 1, for example, a cleaning nozzle 3, an observation window 4a, and an opening 6b of a treatment instrument insertion hole 2a are provided.

Reference numeral 5 denotes a light-emitting device, for example, a light-emitting diode, which is included in a later-described illumination optical portion 20.

As illustrated in Fig. 2, the insertion portion 2 includes a distal end portion 6, a bending portion 7, and a non-illustrated flexible tube portion having flexibility and a tubular shape continuously provided in this order from the distal end side. The bending portion 7 includes a distal end bending piece 7f on the distal end side. On the proximal end side of the distal end bending piece 7f, a bending piece 7a and a plurality of non-illustrated bending pieces are continuously connected so as to bend in, for example, vertical and horizontal directions.

The distal end portion 6 includes a distal end cover 8 and a distal end rigid member 9. The distal end cover 8 includes an insulating member. The distal end rigid member 9 includes a metal member such as stainless steel.

First, the distal end cover 8 will be described.

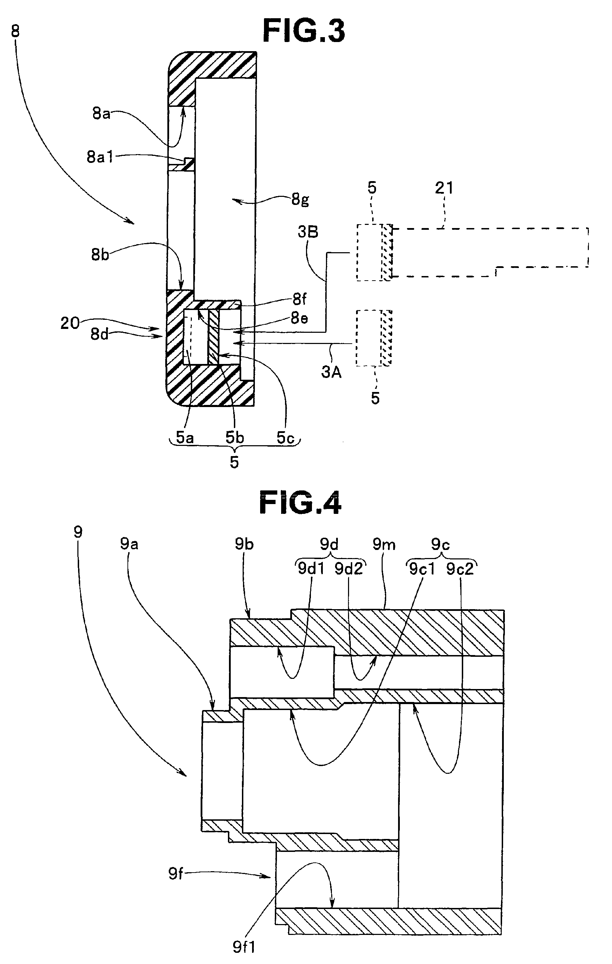

As illustrated in Fig. 3, the distal end cover 8 has a cylindrical shape. The distal end cover 8 is made of, for example, polysulfone, which is a transparent resin member having insulating properties. At a distal end face of the distal end cover 8, a first opening 8a, a second opening 8b, a third opening 8c (see Fig. 1) and an illuminating window portion 8d are provided.

The first opening 8a is a through hole in which the cleaning nozzle 3 is inserted and arranged. The first opening 8a is provided with a convex portion 8a1 that defines a direction of a spout of the cleaning nozzle 3. The second opening 8b is a through hole in which a later-described distal end first convex portion 9a of the distal end rigid member 9 is arranged. The third opening 8c is a through hole included in a treatment instrument insertion hole 2a, which is illustrated in Fig. 1. Center axes (not-illustrated) of the through holes, which are the openings 8a, 8b and 8c, are parallel to a center axis (not illustrated) of the distal end cover 8.

The illuminating window portion 8d includes an exit face that includes a flat face. At a proximal end side of the illuminating window portion 8d, a light-emitting device installing hole 8e in which the light-emitting device 5 is arranged is provided. The illuminating window portion 8d and the light-emitting device 5 fixedly provided in the light-emitting device installing hole 8e are included in the illumination optical portion 20.

The light-emitting device 5 includes a light-emitting portion 5a on the distal end face side and a conductive portion 5b on the proximal end face side. In the present embodiment, as indicated by arrow 3A, the light-emitting device 5 is arranged at a predetermined position in the light-emitting device installing hole 8e and fixed to the light-emitting device installing hole 8e in an integrated manner by bonding.

More specifically, the light-emitting portion 5a faces a surface on the proximal end side of the illuminating window portion 8d. The conductive portion 5b is positioned on the illuminating window portion 8d side of the light-emitting device installing hole 8e. In other words, the conductive portion 5b is covered by a wall portion 8f provided perpendicularly around the conductive portion 5b. Consequently, insulation between the distal end rigid member 9 made of a metal and the conductive portion 5b is reliably ensured and an increase in diameter of the distal end portion 6 is prevented.

The light-emitting device 5 connected to a substrate 21 as indicated by dashed lines may be arranged and fixedly provided in the light-emitting device installing hole 8e of the distal end cover 8 as indicated by arrow 3B. Alternatively, the light-emitting device 5 may be provided at a predetermined position of the distal end cover 8 by means of integral molding. Reference numeral 8g is a distal end rigid member arranging space in which a portion on the distal end side of the distal end rigid member 9 is received.

As illustrated in Fig. 2, a substrate 21 having insulating properties and high thermal conductivity and made of, for example, ceramic is connected to the conductive portion 5b of the light-emitting device 5. The substrate 21 has, for example, a prism shape, and a non-illustrated surface thereof includes a conductive pattern as wirings. Power supply cables 22 and 23 that supply power to the light-emitting device 5 are connected to the conductive pattern. The substrate 21 serves as a heat dissipation member that conducts heat generated by the light-emitting device 5 to the proximal end side, and also as a connecting member that electrically connects the light-emitting device 5 and the power supply cables 22 and 23.

In the present embodiment, the shape of the substrate 21 is not limited to a prism shape, and may be a columnar shape.

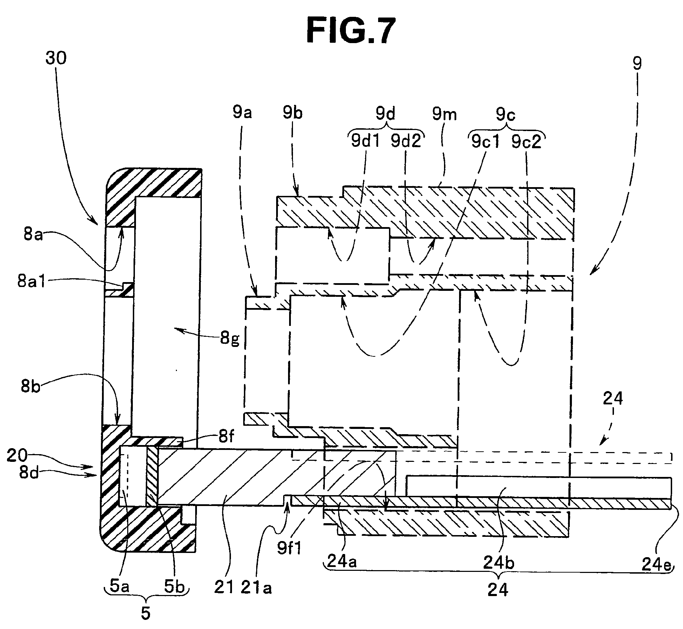

A cutout surface 21a is formed in the substrate 21. On the cutout surface 21a, a heat dissipation plate 24, which is a plate-shaped heat dissipation member, is arranged. The cutout surface 21 a is formed, for example, at a position on the inner surface side of a space 6c inside the distal end portion, the position being away from a center axis of the distal end portion. The heat dissipation plate 24, which is a plate-shaped heat dissipation member, is fixed to the cutout surface 21a in an integrated manner by means of, for example, soldering or brazing. A position where the cutout surface 21a formed in the substrate 21 is arranged is not limited to a position on the inner surface side of the space 6c inside the distal end portion, the position being away from the center axis of the distal end portion. The position where the cutout surface 21a is arranged may be provided on the center axis side of the space 6c inside the distal end portion as indicated by dashed lines in Fig. 7, which will be described later.

The heat dissipation plate 24 is formed of a metal member having high thermal conductivity, such as copper or aluminum. A thickness, a width and a length of the heat dissipation plate 24 are arbitrary determined considering a capacity of heat dissipation (hereinafter referred to as an amount of heat dissipation) and according to the type of the endoscope 1. Also, a cross-sectional shape of the heat dissipation plate 24 may be a desired three-dimensional shape such as an L-shape, a U-shape, a semicircular shape, a concave-shape and a box shape as necessary.

In the figure, the heat dissipation plate 24 is, for example, a flat plate. The heat dissipation plate 24 includes a connection portion 24a and a heat dissipating portion/pipe sleeve 24b. The connection portion 24a is arranged on the cutout surface 21a of the substrate 21 and the heat dissipating portion/pipe sleeve 24b projects from an end face of the substrate 21 toward the proximal end side. A distal end portion of a protection tube 25 inserted in the insertion portion 2, through which the power supply cables 22 and 23 are inserted, is arranged so as to be fitted on the heat dissipating portion/pipe sleeve 24b, and fixed to the heat dissipating portion/pipe sleeve 24b in an integrated manner by, e.g., thread-wrapping bonding.

With such configuration, the heat dissipating portion/pipe sleeve 24b and the power supply cables 22 and 23 are inserted in the protection tube 25, thereby the power supply cables 22 and 23 being stably arranged inside the insertion portion 2 and preventing the heat dissipating portion/pipe sleeve 24b from coming into contact with objects included in the insertion portion. Accordingly, the problems of, e.g., the heat dissipating portion/pipe sleeve 24b damaging the tube or disconnecting a signal line can be solved.

An end portion 24e of the heat dissipation plate 24 is arranged within an endoscope rigid portion. In other words, the end portion 24e is arranged without projecting from the distal end bending piece 7f included in the bending portion 7 included in the insertion portion 2 of the endoscope 1 toward the proximal end side. This is because where the bending portion 7 is subjected to repeated bending operations, the heat dissipation plate 24 is prevented from being deformed due to the bending operations. The endoscope rigid portion is a portion from the distal end face of the distal end cover 8 to a proximal end of the distal end bending piece 7f.

As described above, as a result of the heat dissipation plate 24 being arranged within the endoscope rigid portion, the problem of the heat dissipation plate 24 being damaged as a result of repeated bending of the heat dissipation plate 24 accompanying bending of the bending portion 7 can reliably be prevented.

Meanwhile, the cleaning nozzle 3 and the observation optical portion 10 are provided in the distal end rigid member 9. The observation optical portion 10 includes a lens unit 11 and an image pickup unit 15.

The lens unit 11 includes a lens frame 12 and, for example, optical lenses 4b, 4c, 4d and 4e arranged in the lens frame 12. Meanwhile, the image pickup unit 15 mainly includes an image pickup apparatus 16, and a device frame 17. The lens frame 12 and the device frame 17 are each formed of metal members having high corrosion resistance and low thermal conductivity, such as stainless steel.

The image pickup apparatus 16 includes an image pickup device 16a and a non-illustrated circuit substrate with electronic components mounted thereon. The image pickup device 16a is, e.g., a CCD or a CMOS. For example, two cover lenses 18 and 19, which are optical members, are bonded and thereby fixed to the light receiving face side of the image pickup device 16a. The cover lenses 18 and 19 are arranged in the device frame 17, and the second cover lens 19 is arranged on the light receiving face of the image pickup device 16a. A signal cable 16b including a bundle of signal lines extends out of the image pickup apparatus 16 toward the proximal end side of the insertion portion 2.

As illustrated in Fig. 4, the distal end rigid member 9 includes a distal end first convex portion 9a and a distal end second convex portion 9b. The distal end first convex portion 9a is formed on the distal end side of a component portion body 9m, and is arranged in the second opening 8b. The distal end second convex portion 9b is arranged in the distal end rigid member arranging space 8g. A distal end face of the distal end first convex portion 9a is set so as to project from a distal end face of the distal end second convex portion 9b by a predetermined amount. Consequently, the distal end face of the distal end first convex portion 9a is arranged so as to be aligned with the distal end face of the distal end cover 8 or project relative to the distal end face of the distal end cover 8.

Furthermore, a first through hole 9c, a second through hole 9d, a third through hole 9e and a fourth through hole 9f are provided in the distal end rigid member 9. Center axes (not illustrated) of the respective through holes 9c, 9d, 9e and 9f are parallel to a center axis (not illustrated) of the distal end rigid member 9.

The first through hole 9c is an observation optical portion hole, and includes a lens frame hole 9c1 and a device frame hole 9c2. The lens frame 12 is fixedly provided in the lens frame hole 9c1. The device frame 17 is arranged so as to loosely fit in the device frame hole 9c2.

The second through hole 9d includes a nozzle hole 9d1 and an air/water sending hole 9d2. The cleaning nozzle 3 is fixedly provided in the nozzle hole 9d1. The third through hole 9e is a through hole included in the treatment instrument insertion hole 2a illustrated in Fig. 1.

The fourth through hole 9f is an illumination optical portion hole 9f1. A number of illumination optical portion holes 9f1 are formed, the number being equal to the number of light-emitting devices 5 provided integrally with the distal end cover 8. Each illumination optical portion hole 9f1 is a hole allowing the substrate 21 fixed to the heat dissipation plate 24 in an integrated manner to pass through, and includes a round hole or a rectangular hole.

In the endoscope 1 configured as described above, power is supplied to the light-emitting device 5 in the illumination optical portion 20, which is configured integrally with the distal end cover 8, via the power supply cables 22 and 23 and the light-emitting device 5 thereby emits light. Then, illuminating light emitted from the light-emitting portion 5a passes through the illuminating window portion 8d and illuminates an object to be observed.

In the present embodiment, the distal end face of the distal end first convex portion 9a is arranged so as to be aligned with the distal end face of the distal end cover 8 or project relative to the distal end face of the distal end cover 8. Consequently, it is possible to prevent illuminating light emitted from the light-emitting portion 5a in the light-emitting device 5 and passing through the inside of the distal end cover 8 toward the illuminating window portion 8d from directly entering the observation optical portion 10 via the transparent distal end cover 8.

Also, as a result of the light-emitting device 5 emitting light, heat is generated from the light-emitting device 5. The heat is first conducted to the substrate 21 and then to the connection portion 24a and the heat dissipating portion/pipe sleeve 24b in the heat dissipation plate 24 having a shape with an amount of heat dissipation taken into consideration, and is dissipated into the space 6c inside the distal end portion. Consequently, it is possible to prevent an increase in temperature of the light-emitting device 5, thereby preventing the problem of a decrease in illuminating light amount and also preventing the problem of image noise generated as a result of the heat generated from the light-emitting device 5 being conducted to the image pickup apparatus 16 via the lens frame 12 and the device frame 17 in the observation optical portion 10 provided in the distal end rigid member 9.

In the above-described embodiment, it has been described that the heat dissipation plate 24 is formed so as to have a flat plate shape, and also formed so as to have a desired cross-sectional shape such as an L-shape, a U-shape, a semicircular shape, a concave-channel shape or a box shape as necessary. However, where a capacity of heat generated from the light-emitting device 5 is larger than the amount of heat dissipated by the heat dissipation plate 24, the heat dissipation plate 24 is configured as indicated in a first configuration or a second configuration, which is described below. Consequently, the amount of heat dissipated by the heat dissipation plate 24 can be increased.

In the first configuration, coaxial wires are used instead of the power supply cables 22 and 23. Each coaxial wire includes one core wire and a shield wire provided around the core wire. In such configuration, the shield wire includes, for example, a plurality of copper-based metal elemental wires having high thermal conductivity. In the first configuration, the shield wires in the coaxial wires are connected by, e.g., soldering to the heat dissipating portion/pipe sleeve 24b of the heat dissipation plate 24 as heat dissipation members.

According to the first configuration, heat generated from the light-emitting device 5 is conducted to the substrate 21 and the connection portion 24a and the heat dissipating portion/pipe sleeve 24b of the heat dissipation plate 24 and then conducted to the shield wires and dissipated.

As described above, as a result of shield wires being connected to the heat dissipating portion/pipe sleeve 24b, the amount of heat dissipated by the heat dissipation plate 24 is increased by the shield wires, enabling heat generated from the light-emitting device 5 to be dissipated more reliably.

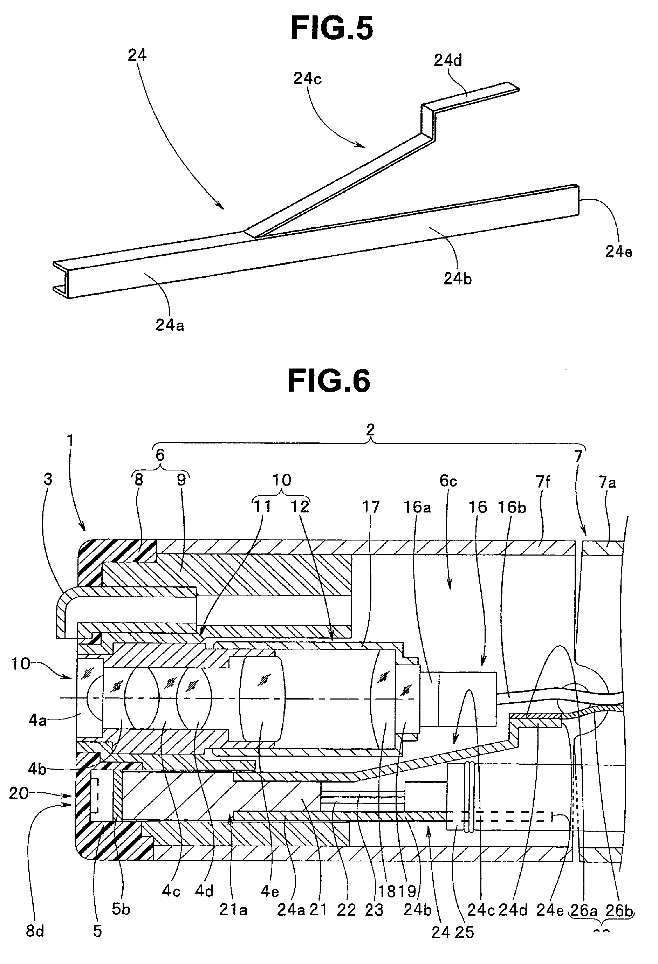

In the second configuration, as illustrated in Fig. 5, the heat dissipation plate 24 is formed so as to have, for example, a concave-shape, and a flexed portion 24c is provided in a part of the heat dissipating portion/pipe sleeve 24b. A proximal end flat face of the flexed portion 24c is a connection and fixing portion 24d to which a flat shape portion 26a, which will be described later, is connected.

As illustrated in Figs. 5 and 6, the flexed portion 24c is arbitrarily flexed so that an end portion 24e thereof is arranged in the vicinity of a center in the distal end bending piece 7f. As a result, the connection and fixing portion 24d is arranged in the vicinity of the center in the distal end bending piece 7f. More specifically, the flat shape portion 26a of a flat braided conductive wire 26, which is a plate-shaped heat dissipation member, is connected to the connection and fixing portion 24d.

The flat braided conductive wire 26 includes metal elemental wires having high thermal conductivity such as copper or aluminum. The flat braided conductive wire 26 includes the flat shape portion 26a and a flat braided wire portion 26b having flexibility. The flat shape portion 26a is hardened by, e.g., soldering so as to have a plate shape. The flat braided wire portion 26b is inserted in a center portion of the insertion portion 2 and extends inside the bending portion 7 toward the proximal end side of the insertion portion.

In the second configuration, heat generated from the light-emitting device 5 is conduced to the substrate 21, and the connection portion 24a and the heat dissipating portion/pipe sleeve 24b of the heat dissipation plate 24 and dissipated into the space 6c inside the distal end portion, and also after the heat is conducted to the substrate 21, and the connection portion 24a, the heat dissipating portion/pipe sleeve 24b and the flexed portion 24c of the heat dissipation plate 24, the heat is further conducted to the flat braided conductive wire 26 and dissipated inside the insertion portion 2.

As described above, the flexed portion 24c is formed in the heat dissipating portion/pipe sleeve 24b, and the flat braided conductive wire 26 is connected to the flexed portion 24c. As a result, the amount of heat dissipated by the heat dissipation plate 24 is increased by the flat braided conductive wire 26, enabling heat generated in the light-emitting device 5 to be dissipated more reliably to the proximal end side of the insertion portion.

Furthermore, the connection and fixing portion 24d is arranged in the vicinity of the center in the distal end bending piece 7f, the flat shape portion 26a of the flat braided conductive wire 26 is connected to the connection and fixing portion 24d, and the flat braided wire portion 26b is arranged so as to be inserted in the center portion of the insertion portion 2. As a result, bendings of the flat braided wire portion 26b accompanying bending operations of the bending portion 7 are substantially decreased, thereby solving the problem of a decrease in heat dissipation property due to disconnection of metal elemental wires.

Alternatively, it is possible that the flat shape portion 26a of the flat braided conductive wire 26 is connected to the cutout surface 21a without the heat dissipation plate 24 being connected to the substrate 21 and the flat braided wire portion 26b is arranged so as to be inserted in the center portion of the insertion portion 2 to dissipate heat generated from the light-emitting device 5.

As illustrated in Fig. 7, the light-emitting device 5, the substrate 21 and the heat dissipation plate 24 are configured integrally with the distal end cover 8 to provide a heat dissipation function-provided cover unit 30 having a heat dissipation function, which is detachably attachable to the distal end rigid member 9.

As described above, the distal end cover 8 is configured as the heat dissipation function-provided cover unit 30, enabling easy assembling of the distal end cover 8 and the distal end rigid member 9 into the distal end portion 6. In other words, the heat dissipation plate 24 and the substrate 21 projecting from the cover unit 30, which are indicated by solid lines in Fig. 7, are inserted into the illumination optical portion hole 9f1 in the distal end rigid member 9, which is indicated by dashed lines, and the distal end second convex portion 9b and the distal end first convex portion 9a, which are indicated by dashed lines, are arranged in the distal end rigid member arranging space 8g and the second opening 8b, which are indicated by solid lines, enabling configuration of the distal end portion 6.

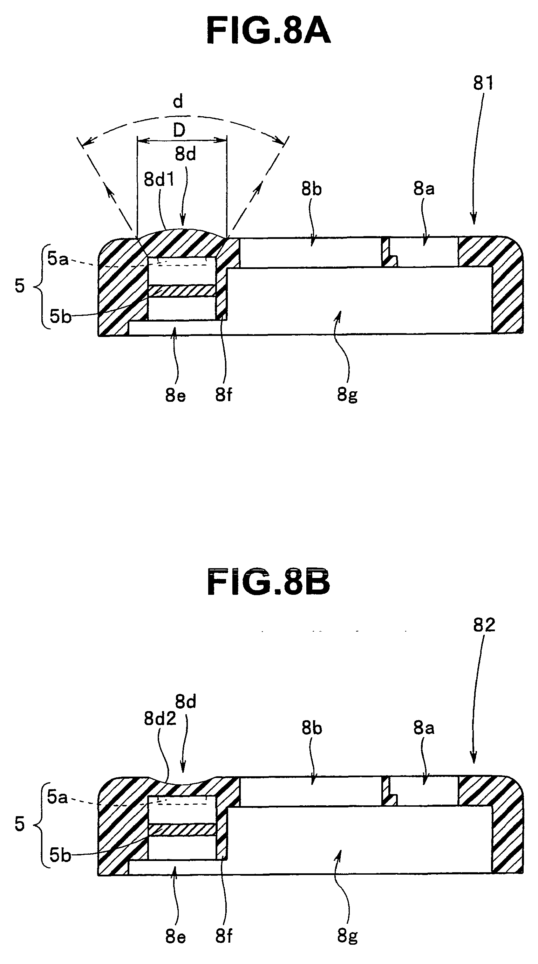

Another example configuration of the distal end cover will be described with referenced to Figs. 8A to 9C.

The distal end cover 8 shown in the present embodiment described above includes the illuminating window portion 8d including an exit face that includes a flat face. However, the exit face of the illuminating window portion 8d provided in the distal end cover 8 is not limited to a flat face, and for example, like a distal end cover 81, which is illustrated in Fig. 8A, the exit face of the illuminating window portion 8d may be an arc-like convex shape portion 8d1.

As describe above, the exit face of the illuminating window portion 8d is formed as the arc-like convex shape portion 8d1 so as to have a lens function. As a result, the problem of illuminating light emitted from the illuminating window portion 8d diffuses around, thereby decreasing the amount of light in the center can be solved.

A convex portion length D of the arc-like convex shape portion 8d1 is at least within a height range d of a light beam emitted from the light-emitting portion 5a. Also, a length of convex in the arc-like convex shape portion 8d1 can be arbitrarily determined as long as the arc-like convex shape portion 8d1 has a structure in which a distal end face of the arc-like convex shape portion 8d1 projects toward the distal end side relative to other distal end faces of the distal end cover 8 and illuminating light emitted from the arc-like convex shape portion 8d1 condenses at the center.

Also, in Fig. 8A, the exit face of the illuminating window portion 8d is the arc-like convex shape portion 8d1. However, as long as the exit face has a structure in which illuminating light emitted from the exit face condenses at the center, the exit face may be the arc-like convex shape portion 8d1 or a polygonal convex shape portion. Alternatively, as long as the exit face has a structure in which illuminating light from the exit face condenses at the center, like a distal end cover 82, which is illustrated in Fig. 8B, the exit face of the illuminating window portion 8d may include an arc-like concave shape portion 8d2 or a polygonal concave shape portion.

In the present embodiment described above, the distal end portion 6 includes the distal end cover 8 including a transparent resin member having insulating properties and the distal end rigid member 9 including a metal member in an integrated manner. Then, the distal end face of the distal end first convex portion 9a of the distal end rigid member 9, which is arranged in the second opening 8b, is arranged so as to be aligned with the distal end face of the distal end cover 8 or project relative to the distal end face of the distal end cover 8. As a result, it is possible to prevent illuminating light emitted from the light-emitting portion 5a of the light-emitting device 5 and passing toward the illuminating window portion 8d from directly entering the observation optical portion 10 via the transparent distal end cover 8.

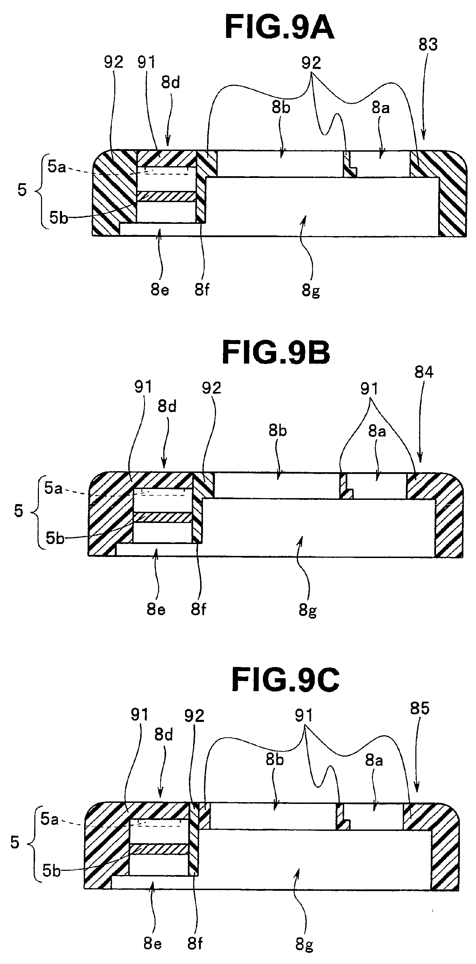

In the distal end portion 6 including the distal end cover 8 and the distal end rigid member 9 in an integrated manner, as illustrated in Figs. 9A, 9B and 9C, the distal end cover 8 includes a transparent first resin member 91 and a colored second resin member 92 that provides a light-blocking portion.

In a distal end cover 83 in Fig. 9A, the illuminating window portion 8d includes the first resin member 91 and all of portions other than the illuminating window portion 8d include the second resin member 92. With the configuration, illuminating light emitted from the light-emitting portion 5a of the light-emitting device 5 passes through the inside of the illuminating window portion 8d that includes the transparent first resin member 91 and is emitted toward an object to be observed. In other words, as a result of the portion around the illuminating window portion 8d and the portion around the observation optical portion 10 including the second resin member 92, illuminating light emitted from the light-emitting portion 5a of the light-emitting device 5 is reliably prevented from directly entering the observation optical portion 10 via the distal end cover 83. Then, the distal end cover 83 is formed by two-color molding using the first resin member 91 and the second resin member 92.

Also, the exit face of the illuminating window portion 8d including the first resin member 91 may be configured as the arc-like convex shape portion 8d1 as illustrated in Fig. 8A or the arc-like concave shape portion 8d2 as illustrated in Fig. 8B.

Also, although not illustrated, a side peripheral face of the illuminating window portion 8d including the first resin member 91 may be formed so as to have a tapered shape with a diameter increasing from the proximal end side toward the distal end side, or an outer diameter of the illuminating window portion 8d including the first resin member 91 may be made to be larger than an outer diameter of the light-emitting device 5.

Furthermore, like a distal end cover 84, which is illustrated in Fig. 9B, it is possible that a portion between the observation optical portion 10 and the illumination optical portion 20 includes the second resin member 92 and all of other portions include the first resin member 91. Alternatively, like a distal end cover 85, which is illustrated in Fig. 9C, it is possible that for example, a half portion on the illumination optical portion 20 side of a portion between the observation optical portion 10 and the illumination optical portion 20 includes the second resin member 92 and all of other portions include the first resin member 91.

Consequently, illuminating light emitted from the light-emitting portion 5a of the light-emitting device 5 can be prevented from directly entering the observation optical portion 10 via the distal end cover 84 or 85. Also, it is possible that for example, a half portion on the observation optical portion 10 side or the entirety of the portion between the observation optical portion 10 and the illumination optical portion 20 of the distal end cover 85 includes the second resin member 92 and all of other portion include the first resin member 91. The distal end covers 84 and 85 are formed by double molding.

The present invention is not limited to the present embodiment described above, and various alternations are possible. The invention is defined by the appended claims.

## Claims

1. An endoscope (1) comprising:
a distal end rigid member (9) made of a metal and provided on a distal end side of a bending portion (7), an observation optical portion (10) being fixedly provided in the distal end rigid member (9);
a distal end cover (8, 81, 82, 83, 84, 85) formed of an insulating resin member and fixed to the distal end rigid member (9) in an integrated manner, the distal end cover (8) including a transparent illuminating window portion (8d) included in an illumination optical portion (20) and a light-emitting device installing hole (8e);
a light-emitting device (5) arranged in the light-emitting device installing hole (8e), the light-emitting device (5) including a light-emitting portion (5a) that faces the illuminating window portion (8d) included in the distal end cover (8, 81, 82, 83, 84, 85) and is fixed at a predetermined position, and a conductive portion (5b) positioned on the illuminating window portion (8d) side relative to an opening of the light-emitting device installing hole (8e), the conductive portion (5b) being opposed to the opening of the light-emitting device installing hole (8e);
a substrate (21) having insulating properties, a cutout surface (21a) and thermal conductance and serving as a heat dissipating member, the substrate (21) being connected to the conductive portion (5b) of the light-emitting device (5) and being adapted to conduct heat generated by the light-emitting device (5) to a proximal end side,
power supply cables (22, 23) that supply power being connected to the substrate (21), wherein the substrate (21) has a surface that includes a conductive pattern as wirings which is connected to the power supply cables (22, 23), and wherein the substrate (21) serves as a connecting member that electrically connects the light-emitting device (5) and the power supply cables (22, 23); and
a heat dissipation member (24) having a plate shape and thermal conductance, an end side (24a) of the heat dissipation member (24) being connected to the cutout surface (21a) of the substrate (21) and another end side (24b) of the heat dissipation member (24) projecting relative to the substrate (21) to dissipate heat conducted to the substrate (21).

2. The endoscope (1) according to claim 1, wherein the light-emitting device (5) is provided in the distal end cover (8) formed of the resin member by integral molding.

3. The endoscope (1) according to claim 1, wherein the plate-shape heat dissipation member (24) is a metal plate member having high thermal conductivity, and an end side of the metal plate member is arranged on the substrate (21) and another end side of the metal plate member is arranged inside a distal end bending piece (7f) included in the bending portion (7).

4. The endoscope (1) according to claim 1,
wherein the plate-shape heat dissipation member (24) is a flat braided conductive wire (26) having flexibility; and
wherein the flat braided conductive wire (26) includes a flat shape portion (26a) arranged on the substrate (21) and a flat braided wire portion (26b) flexed and arranged along a longitudinal center axis of the bending portion (7).

5. The endoscope (1) according to claim 1,
wherein in a configuration in which the distal end cover (81) includes a transparent resin member,
the distal end cover (8) includes a through hole to which a distal end convex portion (8d1) provided in the distal end rigid member (9), the observation optical portion (10) being arranged in the distal end convex portion (8d1), is inserted, and the distal end convex portion (8d1) arranged in the through hole combines as a light-blocking portion that avoids entrance of illuminating light emitted from the light-emitting device (5) to the observation optical portion (10).

6. The endoscope (1) according to claim 1, wherein the distal end cover (83, 84, 85) is formed by double molding using a transparent first resin member (91) that constitutes at least the illuminating window portion (8d), and a colored second resin member (92) that constitutes a light-blocking portion that avoids entrance of illuminating light emitted from the light-emitting device (5) to the observation optical portion (10).

7. The endoscope (1) according to any one of claims 1 through 6,
wherein the light-emitting device (5), the distal end cover (8), the substrate (21) and the heat dissipation member (24) integrally constitute a heat-dissipation function-provided cover unit, and the unit is detachably attachable to the distal end rigid member (9).

## Patentansprüche

1. Ein Endoskop (1) umfassend:
ein steifes, aus Metall hergestelltes Bauteil (9) am distalen Ende, das an einer Seite des distalen Endes eines Biegeabschnitts (7) vorgesehen ist, wobei ein optischer Abschnitt (10) zur Beobachtung an dem steifen Bauteil (9) am distalen Ende fest vorgesehen ist;
eine Abdeckung (8, 81, 82, 83, 84, 85) am distalen Ende, die aus einem isolierenden Harzbauteil gebildet ist und an dem steifen Bauteil (9) am distalen Ende in einer integrierten Weise befestigt ist, wobei die Abdeckung (8) am distalen Ende einen transparenten Beleuchtungsfensterabschnitt (8d), der in einem Beleuchtungsoptikabschnitt (20) enthalten ist, und ein Loch (8e) zur Installation eines lichtemittierenden Geräts einschließt;
ein lichtemittierendes Gerät (5), das in dem Loch (8e) zur Installation eines lichtemittierenden Geräts angeordnet ist, wobei das lichtemittierende Gerät (5) einen lichtemittierenden Abschnitt (5a), der dem transparenten Beleuchtungsfensterabschnitt (8d), der in der Abdeckung (8, 81, 82, 83, 84, 85) am distalen Ende enthalten ist, gegenüberliegt und an einer vorgegebenen Position befestigt ist, und einen leitenden Abschnitt (5b), der auf der Seite des Beleuchtungsfensterabschnitts (8d) relativ zu einer Öffnung des Lochs (8e) zur Installation eines lichtemittierenden Geräts positioniert ist, einschließt, wobei der leitende Abschnitt (5b) der Öffnung des Lochs (8e) zur Installation eines lichtemittierenden Geräts gegenüberliegt;
ein Substrat (21), das isolierende Eigenschaften, eine ausgeschnittene Fläche (21a) und eine thermische Leitfähigkeit aufweist und als ein wärmeableitendes Bauteil dient, wobei das Substrat (21) mit dem leitenden Abschnitt (5b) des lichtemittierenden Geräts (5) verbunden ist und eingerichtet ist, Wärme, die durch das lichtemittierende Gerät (5) erzeugt wird, zu einer Seite am proximalen Ende zu leiten;
Energieversorgungskabel (22, 23), die Energie bereitstellen und mit dem Substrat (21) verbunden sind, wobei das Substrat (21) eine Fläche aufweist, die eine leitende Struktur als Verdrahtung einschließt, die mit den Energieversorgungskabeln (22, 23) verbunden ist, und wobei das Substrat (21) als ein Verbindungsbauteil dient, das das lichtemittierende Gerät (5) und die Energieversorgungskabel (22, 23) elektrisch verbindet; und
ein wärmeableitendes Bauteil (24), das eine plattenförmige Gestalt und eine thermische Leitfähigkeit aufweist, wobei eine Endseite (24a) des wärmeableitenden Bauteils (24) mit der ausgeschnittenen Fläche (21a) des Substrats (21) verbunden ist und eine andere Endseite (24b) des wärmeableitenden Bauteils (24) relativ zu dem Substrat (21) hervorsteht, um Wärme, die zu dem Substrat (21) geleitet wurde, abzuleiten.

2. Das Endoskop (1) gemäß Anspruch 1, wobei das lichtemittierende Gerät (5) in der Abdeckung (8) am distalen Ende, die aus einem isolierenden Harzbauteil gebildet ist, durch integrales Gießen vorgesehen ist.

3. Das Endoskop (1) gemäß Anspruch 1, wobei das plattenförmige wärmeableitende Bauteil (24) ein Metallplattenbauteil mit einer hohen thermischen Leitfähigkeit ist, und eine Endseite des Metallplattenbauteils auf dem Substrat (21) angeordnet ist und eine andere Endseite des Metallplattenbauteils innerhalb eines Biegeteils (7f) am distalen Ende, das in dem Biegeabschnitt (7) enthalten ist, angeordnet ist.

4. Das Endoskop (1) gemäß Anspruch 1,
wobei das plattenförmige wärmeableitende Bauteil (24) ein flacher, geflochtener, leitender Draht (26), der eine Flexibilität aufweist, ist; und
wobei der flache, geflochtene, leitende Draht (26) einen Abschnitt (26a) mit flacher Gestalt, der auf dem Substrat (21) angeordnet ist, und einen flachen, geflochtenen Drahtabschnitt (26b), der gebogen ist und entlang einer zentralen Längsachse des Biegeabschnitts (7) angeordnet ist, einschließt.

5. Das Endoskop (1) gemäß Anspruch 1,
wobei in einer Konfiguration, in der die Abdeckung (81) am distalen Ende ein transparentes Harzbauteil einschließt,
die Abdeckung (8) am distalen Ende eine Durchgangsbohrung, in die ein konvexer Abschnitt (8d1) am distalen Ende, der in dem steife Bauteil (9) am distalen Ende vorgesehen ist, wobei der optischer Abschnitt (10) zur Beobachtung in dem konvexen Abschnitt (8d1) am distalen Ende angeordnet ist, eingeführt ist, einschließt, und wobei der in der Durchgangsbohrung angeordnete konvexe Abschnitt (8d1) am distalen Ende als ein lichtblockierender Abschnitt vereint, der ein Eindringen von illuminierenden Licht, das von dem lichtemittierenden Gerät (5) emittiert wird, in den optischen Abschnitt (10) zur Beobachtung verhindert.

6. Das Endoskop (1) gemäß Anspruch 1, wobei die Abdeckung (83, 84, 85) am distalen Ende durch Doppelgießen unter Verwendung eines transparenten ersten Harzbauteils (91), das zumindest den transparenten Beleuchtungsfensterabschnitt (8d) ausmacht, und ein farbiges zweites Harzbauteil (92), das einen lichtblockierenden Abschnitt, der ein Eindringen von illuminierenden Licht, das von dem lichtemittierenden Gerät (5) emittiert wird, in den optischen Abschnitt (10) zur Beobachtung verhindert, ausmacht, gebildet ist.

7. Das Endoskop (1) gemäß einem der Ansprüche 1 bis 6,
wobei das lichtemittierende Gerät (5), die Abdeckung (8) am distalen Ende, das Substrat (21) und das wärmeableitende Bauteil (24) zusammen eine eine wärmeableitende Funktion bereitstellende Abdeckeinheit ausmachen, und die Einheit abnehmbar an dem steifen Element (9) am distalen Ende angebracht ist.

## Revendications

1. Endoscope (1) comprenant :
un élément rigide d'extrémité distale (9) composé de métal et prévu sur un côté d'extrémité distale d'une partie de courbure (7), une partie optique d'observation (10) étant prévue de manière fixe dans l'élément rigide d'extrémité distale (9) ;
un couvercle d'extrémité distale (8, 81, 82, 83, 84, 85) formé d'un élément en résine isolante et fixé à l'élément rigide d'extrémité distale (9) de manière intégrée, le couvercle d'extrémité distale (8) comprenant une partie de fenêtre d'éclairage transparente (8d) contenue dans une partie optique d'éclairage (20) et un trou (8e) d'installation de dispositif d'émission de lumière ;
un dispositif d'émission de lumière (5) agencé dans le trou (8e) d'installation de dispositif d'émission de lumière, le dispositif d'émission de lumière (5) comprenant une partie d'émission de lumière (5a) qui est en regard de la partie de fenêtre d'éclairage (8d) contenue dans le couvercle d'extrémité distale (8, 81, 82, 83, 84, 85) et est fixée au niveau d'une position prédéterminée, et une partie conductrice (5b) positionnée sur le côté de la partie de fenêtre d'éclairage (8d) par rapport à une ouverture du trou (8e) d'installation de dispositif d'émission de lumière, la partie conductrice (5b) étant opposée à l'ouverture du trou (8e) d'installation de dispositif d'émission de lumière ;
un substrat (21) possédant des propriétés isolantes, une surface de découpe (21a) et une conductance thermique et servant d'élément de dissipation de chaleur, le substrat (21) étant connecté à la partie conductrice (5b) du dispositif d'émission de lumière (5) et étant adapté pour conduire la chaleur générée par le dispositif d'émission de lumière (5) vers un côté d'extrémité proximale,
des câbles d'alimentation (22, 23) qui délivrent la puissance en étant connectés au substrat (21), dans lequel le substrat (21) présente une surface qui comprend un tracé conducteur en tant que câblage qui est connecté aux câbles d'alimentation (22, 23), et dans lequel le substrat (21) sert d'élément de connexion qui connecte électriquement le dispositif d'émission de lumière (5) et les câbles d'alimentation (22, 23) ; et
un élément de dissipation de chaleur (24) présentant une forme de plaque et une conductance thermique, un côté d'extrémité (24a) de l'élément de dissipation de chaleur (24) étant connecté à la surface de découpe (21a) du substrat (21) et un autre côté d'extrémité (24b) de l'élément de dissipation de chaleur (24) faisant saillie par rapport au substrat (21) pour dissiper la chaleur conduite vers le substrat (21).

2. Endoscope (1) selon la revendication 1, dans lequel le dispositif d'émission de lumière (5) est prévu dans le couvercle d'extrémité distale (8) formé de l'élément en résine par moulage intégral.

3. Endoscope (1) selon la revendication 1, dans lequel l'élément de dissipation de chaleur en forme de plaque (24) est un élément de plaque métallique possédant une conductivité thermique élevée, et un côté d'extrémité de l'élément de plaque métallique est agencé sur le substrat (21) et un autre côté d'extrémité de l'élément de plaque métallique est agencé à l'intérieur d'une partie de courbure d'extrémité distale (7f) contenue dans la partie de courbure (7).

4. Endoscope (1) selon la revendication 1,
dans lequel l'élément de dissipation de chaleur en forme de plaque (24) est un câble plat tressé conducteur (26) possédant une flexibilité ; et
dans lequel le câble plat tressé conducteur (26) comprend une partie de forme plate (26a) agencée sur le substrat (21) et une partie de câble plat tressé (26b) fléchie et agencée le long d'un axe central longitudinal de la partie de courbure (7).

5. Endoscope (1) selon la revendication 1,
dans lequel dans une configuration dans laquelle le couvercle d'extrémité distale (81) comprend un élément en résine transparente,
le couvercle d'extrémité distale (8) comprend un trou traversant dans lequel une partie convexe d'extrémité distale (8d1) prévue dans l'élément rigide d'extrémité distale (9), la partie optique d'observation (10) étant agencée dans la partie convexe d'extrémité distale (8d1), est insérée, et la partie convexe d'extrémité distale (8d1) agencée dans le trou traversant combine une partie de blocage de lumière qui évite l'entrée de la lumière d'éclairage émise depuis le dispositif d'émission de lumière (5) vers la partie optique d'observation (10).

6. Endoscope (1) selon la revendication 1, dans lequel le couvercle d'extrémité distale (83, 84, 85) est formé par double moulage en utilisant un premier élément en résine transparente (91) qui constitue au moins la partie de fenêtre d'éclairage (8d), et un deuxième élément en résine colorée (92) qui constitue une partie de blocage de lumière qui évite l'entrée de la lumière d'éclairage émise depuis le dispositif d'émission de lumière (5) vers la partie optique d'observation (10).

7. Endoscope (1) selon l'une quelconque des revendications 1 à 6,
dans lequel le dispositif d'émission de lumière (5), le couvercle d'extrémité distale (8), le substrat (21) et l'élément de dissipation de chaleur (24) constituent solidairement une unité de couvercle pourvue d'une fonction de dissipation de chaleur, et l'unité est attachable de manière détachable à l'élément rigide d'extrémité distale (9).
